# EUROPEAN PATENT SPECIFICATION

(11) **EP 1 562 610 B1**
(45) Date of publication and mention of the grant of the patent: **13.09.2006**
(21) Application number: 03758296.2
(22) Date of filing: 06.10.2003
(51) Int. Cl.: A61K 31/661, A61K 31/7068, A61P 35/00

(54) **COMBINATION THERAPY WITH GEMCITABINE AND ZD6126**
KOMBINATIONSTHERAPIE MIT GEMZITABIN UND ZD6126
POLYTHERAPIE FAISANT APPEL A GEMCITABINE ET A ZD6126

(30) Priority: 09.10.2002 GB 0223379
(43) Date of publication of application: 17.08.2005
(73) Proprietor: Angiogene Pharmaceuticals Limited, Oxford OX4 4GA (GB)
(72) Inventor: BARGE, Alan, Macclesfield, Cheshire SK10 4TG (GB)
(74) Representative: Doble, Richard George Vivian
(86) International application number: PCT/GB2003/004328
(87) International publication number: WO 2004/032939

(56) References cited:
- WO-A-01/74368
- WO-A-99/02166

## Description

The present invention relates to the production of a vascular damaging effect on neovascularization associated with a disease state in a warm-blooded animal such as a human which is optionally being treated with ionising radiation, particularly by the treatment of a cancer involving a solid tumour, which comprises the administration of ZD6126 in combination with gemcitabine; to a pharmaceutical composition comprising ZD6126 and gemcitabine; to a combination product comprising ZD6126 and gemcitabine for use in a method of treatment of a human or animal body by therapy; to a kit comprising ZD6126 and gemcitabine; and to the use of ZD6126 and gemcitabine in the manufacture of a medicament for use in the production of a vascular damaging effect on neovascularization associated with a disease state in a warm-blooded animal such as a human which is optionally being treated with ionising radiation.

Normal angiogenesis plays an important role in a variety of processes including embryonic development, wound healing and several components of female reproductive function. Undesirable or pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacol. Sci. 16: 57-66; Folkman, 1995, Nature Medicine 1: 27-31). Formation of new vasculature by angiogenesis is a key pathological feature of several diseases (J. Folkman, New England Journal of Medicine 333, 1757-1763 (1995)). For example, for a solid tumour to grow it must develop its own blood supply upon which it depends critically for the provision of oxygen and nutrients; if this blood supply is mechanically shut off the tumour undergoes necrotic death. Neovascularisation is also a clinical feature of skin lesions in psoriasis, of the invasive pannus in the joints of rheumatoid arthritis patients and of atherosclerotic plaques. Retinal neovascularisation is pathological in macular degeneration and in diabetic retinopathy.

Reversal of neovascularisation by damaging the newly-formed vascular endothelium is expected to have a beneficial therapeutic effect.

WO 99/02166 describes tricyclic compounds that surprisingly have a selective damaging effect on newly formed vasculature as compared to the normal, established vascular endothelium of the host species. This is a property of value in the treatment of disease states associated with angiogenesis such as cancer, diabetes, psoriasis, rheumatoid arthritis, Kaposi's sarcoma, haemangioma, lymphoedema, acute and chronic nephropathies, atheroma, arterial restenosis, autoimmune diseases, acute inflammation, excessive scar formation and adhesions, endometriosis, dysfunctional uterine bleeding and ocular diseases with retinal vessel proliferation.

Compounds which damage newly formed vasculature are vascular targeting agents (VTAs) and are also known as vascular damaging agents (VDAs).

One such compound described in International Patent Application Publication No. WO 99/02166 is N-acetylcolchinol-O-phosphate, (also know as (5S)-5-(acetylamino)-9,10,11-trimethoxy-6,7-dihydro-5*H*-dibenzo[*a,c*]cyclohepten-3-yl dihydrogen phosphate; Example 1 of WO 99/02166), which is referred to herein as ZD6126:

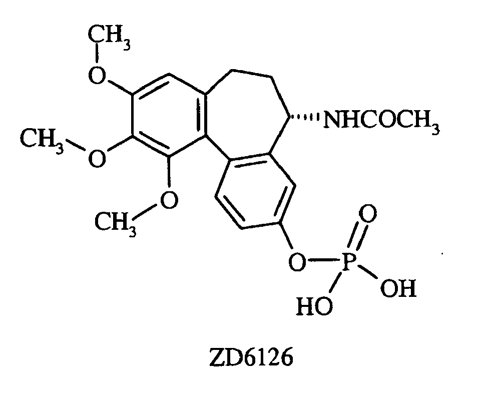

It is believed, though this is not limiting on the invention, that ZD6126 damages newly-formed vasculature, for example the vasculature of tumours, thus effectively reversing the process of angiogenesis. It has been reported that ZD6126 selectively disrupts tumour vasculature leading to vessel occlusion and extensive tumour necrosis (Davis PD, Hill SA, Galbraith SM, et al. Proc. Am Assoc. Cancer Res. 2000; 41: 329).

In WO 99/02166 it is stated that:
"compounds of the invention may be administered as sole therapy or in combination with other treatments. For the treatment of solid tumours compounds of the invention may be administered in combination with radiotherapy or in combination with other anti-tumour substances for example those selected frommitotic inhibitors, for example vinblastine, paclitaxel and docetaxel; alkylating agents, for example cisplatin, carboplatin and cyclophosphamide, antimetabolites, for example 5-fluorouracil, cytosine arabinoside and hydroxyurea; intercalating agents for example adriamycin and bleomycin; enzymes, for example asparaginase; topoisomerase inhibitors for example etoposide, topotecan and irinotecan; thymidylate synthase inhibitors for example raltitrexed; biological response modifers for example interferon; antibodies for example edrecolomab, and anti-hormones for example tamoxifen. Such combination treatment may involve simultaneous or sequential application of the individual components of the treatment."

Nowhere in WO 99/02166 is the specific combination of ZD6126 and gemcitabine suggested.

Nowhere in WO 99/02166 does it state that use of any compound of the invention therein with other treatments will produce surprisingly beneficial effects.

WO 01/74368 discloses the use of ZD6126, or a pharmaceutically acceptable salt thereof, with an effective amount of one of the following therapies: i) ionising radiation; ii) a platinum anti-tumour agent; and iii) a taxane.

Unexpectedly and surprisingly we have now found that the particular compound ZD6126 used in combination with gemcitabine, produces significantly better anti-tumour effects than each of ZD6126 and gemcitabine used alone.

Gemcitabine is (INN) 2'-deoxy-2',2'-difluorocytidine monohydrochloride (β-isomer). Gemcitabine is also known as Gemzar™ (Trademark of Lilly) and it is a cytotoxic agent. It is an anti-metabolite which causes inhibition of DNA synthesis.

Anti-cancer effects of a method of treatment of the present invention include, but are not limited to, anti-tumour effects, the response rate, the time to disease progression and the survival rate. Anti-tumour effects of a method of treatment of the present invention include but are not limited to, inhibition of tumour growth, tumour growth delay, regression of tumour, shrinkage of tumour, increased time to regrowth of tumour on cessation of treatment, slowing of disease progression. It is expected that when a method of treatment of the present invention is administered to a warm-blooded animal such as a human, in need of treatment for cancer involving a solid tumour, said method of treatment will produce an effect, as measured by, for example, one or more of: the extent of the anti-tumour effect, the response rate, the time to disease progression and the survival rate.

According to the present invention there is provided the production of a vascular damaging effect on neovascularization associated with a disease state in a warm-blooded animal such as a human, which comprises administering to said animal an effective amount of ZD6126 or a pharmaceutically acceptable salt thereof, before, after or simultaneously with an effective amount of gemcitabine.

According to a further aspect of the present invention there is provided the production of a vascular damaging effect on neovascularization associated with a disease state in a warm-blooded animal such as a human, which comprises administering to said animal an effective amount of ZD6126 or a pharmaceutically acceptable salt thereof, before, after or simultaneously with an effective amount of gemcitabine, wherein ZD6126 and gemcitabine may each optionally be administered together with a pharmaceutically acceptable excipient or carrier.

According to a further aspect of the present invention there is provided the use of an effective amount of ZD6126 or a pharmaceutically acceptable salt thereof, before, after or simultaneously with an effective amount of gemcitabine, wherein ZD6126 and gemcitabine may each optionally be administered together with a pharmaceutically acceptable excipient or carrier for the manufacture of a medicament for the treatment of a cancer involving a solid tumour in a warm-blooded animal such as a human.

According to a further aspect of the invention there is provided a pharmaceutical composition which comprises ZD6126 or a pharmaceutically acceptable salt thereof, and gemcitabine, in association with a pharmaceutically acceptable excipient or carrier.

According to a further aspect of the present invention there is provided a combination product comprising ZD6126 or a pharmaceutically acceptable salt thereof and gemcitabine, for use in a method of treatment of a human or animal body by therapy.

According to a further aspect of the present invention there is provided a kit comprising ZD6126 or a pharmaceutically acceptable salt thereof, and gemcitabine.

According to a further aspect of the present invention there is provided the use of ZD6126 or a pharmaceutically acceptable salt thereof and gemcitabine for the manufacture of a medicament for the production of a vascular damaging effect on neovascularization associated with a disease a state in a warm-blooded animal such as a human.

According to a further aspect of the present invention there is provided the use of ZD6126 or a pharmaceutically acceptable salt thereof and gemcitabine for the manufacture of a medicament for the production of an anti-cancer effect on neovascularization associated with a disease state in a warm-blooded animal such as a human.

According to a further aspect of the present invention there is provided the use of ZD6126 or a pharmaceutically acceptable salt thereof and gemcitabine for the manufacture of a medicament for the production of an anti-tumour effect in a warm-blooded animal such as a human.

According to the present invention the therapeutic combination treatment comprises the administration of an effective amount of ZD6126 or a pharmaceutically acceptable salt thereof, optionally together with a pharmaceutically acceptable excipient or carrier, and the simultaneous, sequential or separate administration of an effective amount of gemcitabine, wherein gemcitabine may optionally be administered together with a pharmaceutically acceptable excipient or carrier, to a warm-blooded animal such as a human in need of such therapeutic treatment.
Such therapeutic treatment includes a vascular damaging effect, an anti-cancer effect and an anti-tumour effect.

A combination treatment of the present invention as defined herein may be achieved by way of the simultaneous, sequential or separate administration of the individual components of said treatment. A combination treatment as defined herein may be applied as a sole therapy or may involve surgery or radiotherapy or an additional chemotherapeutic agent in addition to a combination treatment of the invention.

Surgery may comprise the step of partial or complete tumour resection, prior to, during or after the administration of the combination treatment with ZD6126 described herein.

Other chemotherapeutic agents for optional use with a combination treatment of the present invention include those described in WO 99/02166 which is incorporated herein by reference; an extract from WO 99/01266 is given hereinbefore. Such chemotherapy may cover five main categories of therapeutic agent:
(i) other antiangiogenic agents including vascular targeting agents;
(ii) cytostatic agents;
(iii) biological response modifiers (for example interferon);
(iv) antibodies (for example edrecolomab); and
(v) antiproliferative/antineoplastic drugs and combinations thereof, as used in medical oncology.

A particular example of a chemotherapeutic agent for use with a combination treatment of the present invention is a platinum anti-cancer agent such as cisplatin; such a combination is expected to be particularly useful for the treatment of lung cancer and bladder cancer.

The administration of a triple combination of ZD6126, gemcitabine and ionising radiation may produce effects, such as anti-tumour effects, greater than those achieved with any of the three therapies used alone, greater than those achieved with the combination of ZD6126 and gemcitabine, greater than those achieved with the combination of ZD6126 and ionising radiation, greater than those achieved with the combination of gemcitabine and ionising radiation.

According to the present invention the production of a vascular damaging effect on neovascularization associated with a disease state in a warm-blooded animal such as a human, comprises administering to said animal an effective amount of ZD6126 or a pharmaceutically acceptable salt thereof, before, after or simultaneously with an effective amount of gemcitabine and before, after or simultaneously with an effective amount of ionising radiation.

According to the present invention the method for the treatment of a cancer involving a solid tumour in a warm-blooded animal such as a human, comprises administering to said animal an effective amount of ZD6126 or a pharmaceutically acceptable salt thereof, before, after or simultaneously with an effective amount of gemcitabine and before, after or simultaneously with an effective amount of ionising radiation.

According to the present invention the production of a vascular damaging effect on neovascularization associated with a disease state a warm-blooded animal such as a human, comprises administering to said animal an effective amount of ZD6126 or a pharmaceutically acceptable salt thereof, before, after or simultaneously with an effective amount of gemcitabine and before, after or simultaneously with an effective amount of ionising radiation, wherein ZD6126 and gemcitabine may each optionally be administered together with a pharmaceutically acceptable excipient or carrier.

According to the present invention the treatment of a cancer involving a solid tumour in a warm-blooded animal such as a human, comprises administering to said animal an effective amount of ZD6126 or a pharmaceutically acceptable salt thereof, before, after or simultaneously with an effective amount of gemcitabine and before, after or simultaneously with an effective amount of ionising radiation, wherein ZD6126 and gemcitabine may each optionally be administered together with a pharmaceutically acceptable excipient or carrier.

According to a further aspect of the present invention there is provided the use of ZD6126 or a pharmaceutically acceptable salt thereof and gemcitabine for the manufacture of a medicament for the production of a vascular damaging effect on neovascularization associated with a disease state in a warm-blooded animal such as a human which is being treated with ionising radiation.

According to a further aspect of the present invention there is provided the use of ZD6126 or a pharmaceutically acceptable salt thereof and gemcitabine for the manufacture of a medicament for the production of an anti-cancer effect in a warm-blooded animal such as a human which is being treated with ionising radiation.

According to a further aspect of the present invention there is provided the use of ZD6126 or a pharmaceutically acceptable salt thereof and gemcitabine for the manufacture of a medicament for the production of an anti-tumour effect in a warm-blooded animal such as a human which is being treated with ionising radiation.

According to the present invention the therapeutic combination treatment comprises the administration of an effective amount of ZD6126 or a pharmaceutically acceptable salt thereof, optionally together with a pharmaceutically acceptable excipient or carrier, and the simultaneous, sequential or separate administration of an effective amount of gemcitabine, optionally together with a pharmaceutically acceptable excipient or carrier and before, after or simultaneously with an effective amount of ionising radiation, to a warm-blooded animal such as a human in need of such therapeutic treatment.

A warm-blooded animal such as a human which is being treated with ionising radiation means a warm-blooded animal such as a human which is treated with ionising radiation before, after or at the same time as the administration of a medicament or combination treatment comprising ZD6126 and gemcitabine. For example said ionising radiation may be given to said warm-blooded animal such as a human within the period of a week before to a week after the administration of a medicament or combination treatment comprising ZD6126 and gemcitabine. This means that ZD6126, gemcitabine and ionising radiation may be administered separately or sequentially in any order, or may be administered simultaneously. The warm-blooded animal may experience the effect of each of ZD6126, gemcitabine and radiation simultaneously.

According to one aspect of the present invention the ionising radiation is administered before one of ZD6126 and gemcitabine or after one of ZD6126 and gemcitabine.

According to another aspect of the present invention the ionising radiation is administered before both ZD6126 and gemcitabine or after both ZD6126 and gemcitabine.

According to another aspect of the present invention ZD6126 is given after the other element(s) of the combination treatment.

According to another aspect of the present invention the effect of a method of treatment of the present invention is expected to be at least equivalent to the addition of the effects of each of the components of said treatment used alone, that is, of each of ZD6126 and gemcitabine used alone or of each of ZD6126, gemcitabine and ionising radiation used alone.

According to another aspect of the present invention the effect of a method of treatment of the present invention is expected to be greater than the addition of the effects of each of the components of said treatment used alone, that is, of each of ZD6126 and gemcitabine used alone or of each of ZD6126, gemcitabine and ionising radiation, used alone.

According to another aspect of the present invention the effect of a method of treatment of the present invention is expected to be a synergistic effect.

According to the present invention a combination treatment is defined as affording a synergistic effect if the effect is therapeutically superior, as measured by, for example, the extent of the response, the response rate, the time to disease progression or the survival period, to that achievable on dosing one or other of the components of the combination treatment at its conventional dose. For example, the effect of the combination treatment is synergistic if the effect is therapeutically superior to the effect achievable with ZD6126 or gemcitabine or ionising radiation alone. Further, the effect of the combination treatment is synergistic if a beneficial effect is obtained in a group of patients that does not respond (or responds poorly) to ZD6126 or gemcitabine or ionising radiation alone. In addition, the effect of the combination treatment is defined as affording a synergistic effect if one of the components is dosed at its conventional dose and the other component(s) is/are dosed at a reduced dose and the therapeutic effect, as measured by, for example, the extent of the response, the response rate, the time to disease progression or the survival period, is equivalent to that achievable on dosing conventional amounts of the components of the combination treatment. In particular, synergy is deemed to be present if the conventional dose of ZD6126 or gemcitabine or ionising radiation may be reduced without detriment to one or more of the extent of the response, the response rate, the time to disease progression and survival data, in particular without detriment to the duration of the response, but with fewer and/or less troublesome side-effects than those that occur when conventional doses of each component are used.

As stated above the combination treatments of the present invention as defined herein are of interest for their vascular damaging effects. Neovascularisation occurs in a wide range of disease states including cancer, (including leukaemia, multiple myeloma and lymphoma), diabetes, psoriasis, rheumatoid arthritis, Kaposi's sarcoma, haemangioma, acute and chronic nephropathies, atheroma, arterial restenosis, autoimmune diseases, acute inflammation, endometriosis, dysfunctional uterine bleeding and ocular diseases with retinal vessel proliferation including age-related macular degeneration. Combination treatments of the present invention are expected to be particularly useful in the prophylaxis and treatment of diseases such as cancer and Kaposi's sarcoma. More particularly such combination treatments of the invention are expected to slow advantageously the growth of primary and recurrent solid tumours of, for example, the colon, pancreas, bladder, breast, prostate, lungs and skin. Especially combination treatments of the present invention are expected to slow advantageously the growth of tumours in pancreatic cancer and lung cancer, for example mesothelioma and non-small cell lung cancer (NSCLC).

The compositions described herein may be in a form suitable for oral administration, for example as a tablet or capsule, for parenteral injection (including intravenous, subcutaneous, intramuscular, intravascular or infusion) for example as a sterile solution, suspension or emulsion, for topical administration for example as an ointment or cream, for rectal administration for example as a suppository or the route of administration may be by direct injection into the tumour or by regional delivery or by local delivery. In other embodiments of the present invention the ZD6126 of the combination treatment may be delivered endoscopically, intratracheally, intralesionally, percutaneously, intravenously, subcutaneously, intraperitoneally or intratumourally. Preferably ZD6126 is administered intravenously. In general the compositions described herein may be prepared in a conventional manner using conventional excipients. The compositions of the present invention are advantageously presented in unit dosage form.

ZD6126 will normally be administered to a warm-blooded animal at a unit dose within the range 10-500mg per square metre body area of the animal, for example approximately 0.3-15mg/kg in a human. A unit dose in the range, for example, 0.3-15mg/kg, preferably 0.5-5mg/kg is envisaged and this is normally a therapeutically-effective dose. A unit dosage form such as a tablet or capsule will usually contain, for example 25-250mg of active ingredient. Preferably a daily dose in the range of 0.5-5mg/kg is employed.

It has been reported, in WO 01/4369, that the effect of a given dose of ZD6126 can be increased by administering it in divided doses. Divided doses, also called split doses, means that the total dose to be administered to a warm-blooded animal, such as a human, in any one day period (for example one 24 hour period from midnight to midnight) is divided up into two or more fractions of the total dose and these fractions are administered with a time period between each fraction of about greater than 0 hours to about 10 hours, preferably about 1 hour to about 6 hours, more preferably about 2 hours to about 4 hours. The fractions of total dose may be about equal or unequal.

For example the total dose may be divided into two parts which may be about equal with a time interval between doses of greater than or equal to two hours and less than or equal to 4 hours.

ZD6126 may be administered in divided doses when used in combination with gemcitabine.

Gemcitabine may be administered according to known clinical practice. For example in NSCLC the recommended dose of gemcitabine is 1000mg/m² given by 30 minute intravenous infusion. This may be repeated once weekly for three weeks, followed by a one week rest period. This four week cycle may then be repeated. Dosage reduction may be necessary if the patient experiences undue toxicity. In pancreatic cancer the recommended dose of gemcitabine is 1000mg/m² given by 30 minute intravenous infusion. This may be repeated once weekly for seven weeks followed by a week of rest. Subsequent cycles may consist of injections once weekly for three consecutive weeks out of every four weeks. Dosage reduction may be necessary if the patient experiences undue toxicity.

The dosages and schedules may vary according to the particular disease state and the overall condition of the patient. Dosages and schedules may also vary if, in addition to a combination treatment of the present invention, one or more additional chemotherapeutic agents is/are used. Scheduling can be determined by the practitioner who is treating any particular patient.

Radiotherapy may be administered according to the known practices in clinical radiotherapy. The dosages of ionising radiation will be those known for use in clinical radiotherapy. The radiation therapy used will include for example the use of γ-rays, X-rays, and/or the directed delivery of radiation from radioisotopes. Other forms of DNA damaging factors are also included in the present invention such as microwaves and UV-irradiation. For example X-rays may be dosed in daily doses of 1.8-2.0Gy, 5 days a week for 5-6 weeks. Normally a total fractionated dose will lie in the range 45-60Gy. Single larger doses, for example 5-10Gy may be administered as part of a course of radiotherapy. Single doses may be administered intraoperatively. Hyperfractionated radiotherapy may be used whereby small doses of X-rays are administered regularly over a period of time, for example 0.1Gy per hour over a number of days. Dosage ranges for radioisotopes vary widely, and depend on the half-life of the isotope, the strength and type of radiation emitted, and on the uptake by cells.

As stated above the size of the dose of each therapy which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the above-mentioned doses of the components of the combination treatment in order to reduce toxicity.

The present invention relates to combinations of gemcitabine with ZD6126 or a salt thereof. Salts of ZD6126 for use in pharmaceutical compositions will be pharmaceutically acceptable salts, but other salts may be useful in the production of ZD6126 and its pharmaceutically acceptable salts. Such salts may be formed with an inorganic or organic base which affords a pharmaceutically acceptable cation. Such salts with inorganic or organic bases include for example an alkali metal salt, such as a sodium or potassium salt, an alkaline earth metal salt such as a calcium or magnesium salt, an ammonium salt or for example a salt with methylamine, dimethylamine, trimethylamine, piperidine, morpholine or tris-(2-hydroxyethyl)amine.

Gemcitabine is commercially available.

ZD6126 may be made according to the following process.

N-Acetylcolchinol (30.0g, 83.9mmol) is dissolved in acetonitrile under an inert atmosphere and 1,2,3-triazole (14.67g, 212.4mmol) added via a syringe. Di-tert-butyldiethylphosphoramidite (37.7g, 151.4mmol) is added and the reaction mixture stirred at about 20°C to complete the formation of the intermediate phosphite ester. Cumene hydroperoxide (24.4g, 159.2mmol) is added at about 10°C and the reaction mixture stirred until the oxidation is complete. Butyl acetate (50ml) and sodium hydroxide solution (250ml of 1M) are added, the reaction mixture stirred and the aqueous phase discarded. The organic solution is washed with sodium hydroxide solution (2 x 250ml of 1M) and a saturated solution of sodium chloride. Trifluoroacetic acid (95.3g, 836mmol) is added at about 15°C. The reaction mixture is distilled at atmospheric pressure, ZD6126 crystallises and is isolated at ambient temperature.

The following tests may be used to demonstrate the activity of ZD6126 in combination with gemcitabine.

### Model to investigate effects of ZD6126 and gemcitabine therapy on primary pancreatic tumour growth and metastases growing orthotopically in nude mice

Nude mice (n=8 to 10 per group) were treated 9 days after injection of 1x10⁶ L3.6pl cells into the pancreas, with gemcitabine alone (100mg/kg by intraperitoneal injection (i.p.) twice weekly on treatment days 2, 5, 9, 12), ZD6126 alone (75mg/kg i.p. daily for 5 days per week on treatment days 1-5 and 8-12), or a combination of both agents ie gemcitabine (100mg/kg i.p. twice weekly) and ZD6126 (75mg/kg i.p. daily for 5 days per week). Thus the combination group received ZD6126 on treatment days 1-5 and 8-12 and gemcitabine on treatment days 2 and 5 and 9 and 12. On the days where both agents were given, gemcitabine was dosed at least 1 hour before ZD6126. A control group of 10 mice received intraperitoneal injections of an equivalent volume of saline according to the same treatment schedule as the combination group. The animals were sacrificed 25 days after tumour cell injection.

The incidence of pancreatic tumours, liver metastases, lymph node metastases and peritoneal carcinosis was evaluated. The primary pancreatic tumour weight was measured. All macroscopically enlarged lymph nodes and liver nodules were confirmed by histopathology (H&E staining).
The results are shown in Table 1

**Table 1**

| Therapy Group | Incidence Pancreas | Liver Met | LN Met | Peritoneal Carcinosis | Tumour Weight Average +/- std dev | Body Weight Average +/- std dev |
|---|---|---|---|---|---|---|
| | | | | | mg | g |
| Control | 10/10 | 6/10 | 10/10 | 7/10 | 1320 +/- 297 | 19.8 +/- 3.7 |
| ZD6126 | 8/8 | 1/8 | 2/8 | 0/8 | 541 +/- 201 | 18.5 +/- 1.9 |
| Gemcitabine | 10/10 | 2/10 | 10/10 | 1/10 | 687 +/- 157 | 19.1 +/- 2.2 |
| ZD6126 + Gemcitabine | 8/8 | 1/8 | 3/8 | 0/8 | 443 +/- 61 | 17.0+/-2.0 |

Compared with the average weight of control tumours (1320mg), tumours in treated animals reached an average weight of 687mg (gemcitabine) 541mg (ZD6126) and 443mg (gemcitabine + ZD6126). Lymph node metastases were present in 10/10 control and 10/10 gemcitabine treated animals, but only 2/8 and 3/8 animals on ZD6126 or combination treatment displayed lymph node metastases, respectively. Peritoneal carcinosis was present in 7/10 control animals but in 0/8 animals treated with either ZD6126 alone or ZD6126 and gemcitabine.

## Claims

1. A pharmaceutical composition which comprises ZD6126 or a pharmaceutically acceptable salt thereof, and gemcitabine, in association with a pharmaceutically acceptable excipient or carrier.

2. A kit comprising ZD6126 or a pharmaceutically acceptable salt thereof, and gemcitabine.

3. Use of ZD6126 or a pharmaceutically acceptable salt thereof, in combination with gemcitabine, for the manufacture of a medicament for the production of a vascular damaging effect on neovascularisation associated with a disease state in a warm-blooded animal such as a human.

4. Use of ZD6126 or a pharmaceutically acceptable salt thereof, in combination with gemcitabine for the manufacture of a medicament for the production of a vascular damaging effect on neovascularisation associated with a disease state in a warm-blooded animal such as a human which is being treated with ionising radiation.

5. Use of ZD6126 or a pharmaceutically acceptable salt thereof, in combination with gemcitabine for the manufacture of a medicament for the production of an anti-cancer effect in a warm-blooded animal such as a human.

6. Use of ZD6126 or a pharmaceutically acceptable salt thereof, in combination with gemcitabine for the manufacture of a medicament for the production of an anti-cancer effect in a warm-blooded animal such as a human which is being treated with ionising radiation.

7. Use of ZD6126 or a pharmaceutically acceptable salt thereof, in combination with gemcitabine for the manufacture of a medicament for the production of an anti-tumour effect in a warm-blooded animal such as a human.

8. Use of ZD6126 or a pharmaceutically acceptable salt thereof, in combination with gemcitabine for the manufacture of a medicament for the production of an anti-tumour effect in a warm-blooded animal such as a human which is being treated with ionising radiation.

9. Use of ZD6126 or a pharmaceutically acceptable salt thereof, in combination with gemcitabine for the manufacture of a medicament for the treatment of pancreatic cancer or lung cancer in a warm-blooded animal such as a human.

10. Use of ZD6126 or a pharmaceutically acceptable salt thereof, in combination with gemeitabine for the manufacture of a medicament for the treatment of pancreatic cancer or lung cancer in a warm-blooded animal such as a human which is being treated with ionising radiation.

11. Use of ZD6126 or a pharmaceutically acceptable salt thereof, in combination with gemcitabine and a platinum anti-cancer agent for the manufacture of a medicament for the production of an anti-cancer effect in a warm-blooded animal such as a human.

12. Use of ZD6126 or a pharmaceutically acceptable salt thereof, in combination with gemcitabine and a platinum anti-cancer agent for the manufacture of a medicament for the production of an anti-cancer effect in a warm-blooded animal such as a human which is being treated with ionising radiation.

13. The use according to any one of claims 3 to 12 wherein the wherein the total dose of the ZD6126 or a pharmaceutically acceptable salt thereof, to be administered in any one day period, is to be administered to the warm-blooded animal in divided doses.

## Patentansprüche

1. Pharmazeutische Zusammensetzung, enthaltend ZD6126 oder ein pharmazeutisch annehmbares Salz davon und Gemcitabin zusammen mit einem pharmazeutisch annehmbaren Hilfsstoff oder Träger.

2. Kit, enthaltend ZD6126 oder ein pharmazeutisch annehmbares Salz davon und Gemcitabin.

3. Verwendung von ZD6126 oder einem pharmazeutisch annehmbaren Salz davon in Kombination mit Gemcitabin zur Herstellung eines Arzneimittels zur Erzielung einer gefäßschädigenden Wirkung auf die mit einem Krankheitszustand assoziierte Neovaskularisation bei einem Warmblüter wie dem Menschen.

4. Verwendung von ZD6126 oder einem pharmazeutisch annehmbaren Salz davon in Kombination mit Gemcitabin zur Herstellung eines Arzneimittels zur Erzielung einer gefäßschädigenden Wirkung auf die mit einem Krankheitszustand assoziierte Neovaskularisation bei einem Warmblüter wie dem Menschen, der mit ionisierender Strahlung behandelt wird.

5. Verwendung von ZD6126 oder einem pharmazeutisch annehmbaren Salz davon in Kombination mit Gemcitabin zur Herstellung eines Arzneimittels zur Erzielung einer Antikrebswirkung bei einem Warmblüter wie dem Menschen.

6. Verwendung von ZD6126 oder einem pharmazeutisch annehmbaren Salz davon in Kombination mit Gemcitabin zur Herstellung eines Arzneimittels zur Erzielung einer Antikrebswirkung bei einem Warmblüter wie dem Menschen, der mit ionisierender Strahlung behandelt wird.

7. Verwendung von ZD6126 oder einem pharmazeutisch annehmbaren Salz davon in Kombination mit Gemcitabin zur Herstellung eines Arzneimittels zur Erzielung einer Antitumorwirkung bei einem Warmblüter wie dem Menschen.

8. Verwendung von ZD6126 oder einem pharmazeutisch annehmbaren Salz davon in Kombination mit Gemcitabin zur Herstellung eines Arzneimittels zur Erzielung einer Antitumorwirkung bei einem Warmblüter wie dem Menschen, der mit ionisierender Strahlung behandelt wird.

9. Verwendung von ZD6126 oder einem pharmazeutisch annehmbaren Salz davon in Kombination mit Gemcitabin zur Herstellung eines Arzneimittels zur Behandlung von Pankreaskrebs oder Lungenkrebs bei einem Warmblüter wie dem Menschen.

10. Verwendung von ZD6126 oder einem pharmazeutisch annehmbaren Salz davon in Kombination mit Gemcitabin zur Herstellung eines Arzneimittels zur Behandlung von Pankreaskrebs oder Lungenkrebs bei einem Warmblüter wie dem Menschen, der mit ionisierender Strahlung behandelt wird.

11. Verwendung von ZD6126 oder einem pharmazeutisch annehmbaren Salz davon in Kombination mit Gemcitabin und einem Platin-Antikrebsmittel zur Herstellung eines Arzneimittels zur Erzielung einer Antikrebswirkung bei einem Warmblüter wie dem Menschen.

12. Verwendung von ZD6126 oder einem pharmazeutisch annehmbaren Salz davon in Kombination mit Gemcitabin und einem Platin-Antikrebsmittel zur Herstellung eines Arzneimittels zur Erzielung einer Antikrebswirkung bei einem Warmblüter wie dem Menschen, der mit ionisierender Strahlung behandelt wird.

13. Verwendung nach einem der Ansprüche 3 bis 12, bei dem die im Laufe eines Tages zu verabreichende Gesamtdosis des ZD6126 oder eines pharmazeutisch annehmbaren Salzes davon dem Warmblüter in Teildosen zu verabreichen ist.

## Revendications

1. Composition pharmaceutique comprenant du ZD6126 ou un sel pharmaceutiquement acceptable de celui-ci, et de la gemcitabine, en association avec un excipient ou un support pharmaceutiquement acceptable.

2. Kit comprenant du ZD6126 ou un sel pharmaceutiquement acceptable de celui-ci, et de la gemcitabine.

3. Utilisation du ZD6126 ou d'un sel pharmaceutiquement acceptable de celui-ci, en combinaison avec de la gemcitabine, pour la fabrication d'un médicament destiné à la production d'un effet d'endommagement vasculaire sur la néovascularisation associée à un état maladif chez un animal à sang chaud, tel qu'un être humain.

4. Utilisation du ZD6126 ou d'un sel pharmaceutiquement acceptable de celui-ci, en combinaison avec de la gemcitabine, pour la fabrication d'un médicament destiné à la production d'un effet d'endommagement vasculaire sur la néovascularisation associée à un état maladif chez un animal à sang chaud, tel qu'un être humain qui est traité par un rayonnement ionisant.

5. Utilisation du ZD6126 ou d'un sel pharmaceutiquement acceptable de celui-ci, en combinaison avec de la gemcitabine, pour la fabrication d'un médicament destiné à la production d'un effet anti-cancéreux chez un animal à sang chaud, tel qu'un être humain.

6. Utilisation du ZD6126 ou d'un sel pharmaceutiquement acceptable de celui-ci, en combinaison avec de la gemcitabine, pour la fabrication d'un médicament destiné à la production d'un effet anti-cancéreux chez un animal à sang chaud, tel qu'un être humain qui est traité par un rayonnement ionisant.

7. Utilisation du ZD6126 ou d'un sel pharmaceutiquement acceptable de celui-ci, en combinaison avec de la gemcitabine, pour la fabrication d'un médicament destiné à la production d'un effet anti-tumoral chez un animal à sang chaud, tel qu'un être humain.

8. Utilisation du ZD6126 ou d'un sel pharmaceutiquement acceptable de celui-ci, en combinaison avec de la gemcitabine, pour la fabrication d'un médicament destiné à la production d'un effet anti-tumoral chez un animal à sang chaud, tel qu'un être humain qui est traité par un rayonnement ionisant.

9. Utilisation du ZD6126 ou d'un sel pharmaceutiquement acceptable de celui-ci, en combinaison avec de la gemcitabine, pour la fabrication d'un médicament destiné au traitement du cancer du pancréas ou du cancer du poumon chez un animal à sang chaud tel qu'un être humain.

10. Utilisation du ZD6126 ou d'un sel pharmaceutiquement acceptable de celui-ci, en combinaison avec de la gemcitabine, pour la fabrication d'un médicament destiné au traitement du cancer du pancréas ou du cancer du poumon chez un animal à sang chaud tel qu'un être humain qui est traité par un rayonnement ionisant.

11. Utilisation du ZD6126 ou d'un sel pharmaceutiquement acceptable de celui-ci, en combinaison avec de la gemcitabine et un agent anti-cancéreux au platine, pour la fabrication d'un médicament destiné à la production d'un effet anti-cancéreux chez un animal à sang chaud, tel qu'un être humain.

12. Utilisation du ZD6126 ou d'un sel pharmaceutiquement acceptable de celui-ci, en combinaison avec de la gemcitabine et un agent anti-cancéreux au platine, pour la fabrication d'un médicament destiné à la production d'un effet anti-cancéreux chez un animal à sang chaud, tel qu'un être humain qui est traité par un rayonnement ionisant.

13. Utilisation selon l'une quelconque des revendications 3 à 12, dans laquelle la dose totale du ZD6126 ou d'un sel pharmaceutiquement acceptable de celui-ci, destinée à être administrée sur une période quelconque d'une journée, doit être administrée à l'animal à sang chaud en doses fractionnées.
